# EUROPEAN PATENT SPECIFICATION

(11) **EP 2 962 098 B1**
(45) Date of publication and mention of the grant of the patent: **22.03.2017**
(21) Application number: 14714368.9
(22) Date of filing: 25.02.2014
(51) Int. Cl.: G01N 33/12, G01N 33/04, G01N 1/40, G01N 1/22

(54) **DEVICE FOR SAMPLING FOOD PRODUCTS**
VORRICHTUNG ZUR ENTNAHME VON NAHRUNGSMITTELN
DISPOSITIF D'ÉCHANTILLONNAGE DE PRODUITS ALIMENTAIRES

(30) Priority: 28.02.2013 IT RM20130117
(43) Date of publication of application: 06.01.2016
(73) Proprietor: Università Campus Bio-Medico di Roma, 00128 Roma (RM) (IT)
(72) Inventor: PENNAZZA, Giorgio, I-00174 Rome (IT); SANTONICO, Marco, I-00173 Rome (IT); ZOMPANTI, Alessandro, I-03100 Frosinone (IT); DACHÀ, Marina, I-00187 Rome (IT); D'AMICO, Arnaldo, I-00178 Rome (IT)
(74) Representative: Papa, Elisabetta
(86) International application number: PCT/IB2014/059226
(87) International publication number: WO 2014/132185

(56) References cited:
- WO-A1-96/29587
- WO-A2-2006/043297
- US-A1- 2009 199 621
- RUIZ JORGE ET AL: "New device for direct extraction of volatiles in solid samples using SPME", JOURNAL OF AGRICULTURAL AND FOOD CHEMISTRY, AMERICAN CHEMICAL SOCIETY, US, vol. 49, no. 11, 1 January 2001 (2001-01-01), pages 5115-5121, XP002509124, ISSN: 0021-8561, DOI: 10.1021/JF0101298 [retrieved on 2001-10-27]
- TADEUSZ GÓRECKI ET AL: "Theory of analyte extraction by selected porous polymer SPME fibres+", THE ANALYST, vol. 124, no. 5, 1 January 1999 (1999-01-01), pages 643-649, XP055116699, ISSN: 0003-2654, DOI: 10.1039/a808487d

## Description

### Technical field of the invention

The present invention relates to a device and a method for sampling volatile composites from a food product, particularly suitable for checking the maturing of the raw ham.

### Background

In the field of the food industry, the sample phase has a fundamental role in checking quality.

Such sampling results to be particularly important for checking the maturing of products such as for example cheese or raw ham. As far as specifically the latter product and the similar ones are concerned, the check of the maturing product is traditionally performed by means of inserting a punch-shaped element in the area surrounding the femur proximal epiphysis. The above-mentioned punch-shaped element, in the specific case of the raw ham, is constituted by a highly porous material - in particular, typically obtained from a horse bone - which succeeds in absorbing the volatile chemical substances contained inside the thigh. As soon as extracted, the punch-shaped element is sniffed by a "*panelist*" which evaluates the smell thereof, as the volatile compounds released by the punch-shaped element provide indeed information relating the maturing level and the quality of the sample.

The just-described sampling modes result to be intrinsically subjective, as they are linked to the sensitivity of the *panelist.*

Ruiz et al ("New device for direct extraction of volatiles in solid samples using SPME", Journal of Agricultural and Food Chemistry, American Chemical Society, Vol. 49, Nr. 11, 01.01.2011, pages 51115-5121) describe a device for extracting the volatile substances in solid food and for the subsequent analysis by means of SPME. The device has a body bearing an end suitable to penetrate the food product. Within such body a needle can be receive, housing inside a fibre for SPME and which, in turn, is supported by a "SPME holder". Before the insertion into the food product, needle and fibre are indeed both received in an upper longitudinal seat of the main body. After the insertion of the device in the food product, the fibre is made to protrude from the needle, so that it arranges within an enlarged lower chamber of the main body. Such enlarged chamber, on its own side coating, has through-holes suitable to make the volatile substances to be analyzed to penetrate therein, which then come into contact with the fibre. Once a sampling time has elapsed, the fibre and the needle are extracted from the device and inserted in a measuring apparatus.

US 2009/199621 describes a device for collecting a sample of product for a subsequent SPME analysis. Such device comprises a "SPME holder" which is arranged adjacent a sample container. The holder, inside thereof, slidingly supports a cylinder, wherein a piston is slidingly received, the latter integral to a rod bearing a fibre. The fibre, in turn, is received within a needle integral to the cylinder. Upon use, the cylinder slides within the holder integrally to the piston to penetrate the container. At this point, the piston is actuated to expose the fibre in the collecting environment, outside the needle.

None of the above-mentioned documents provides a device for sampling volatile substances compatible with the different analyses from the SPME one. Furthermore, the described analyses have a complex and intrinsically little robust structure, also due to the need for shielding the fibre during the phase of inserting into the product to be sampled.

### Summary of the invention

The technical problem placed and solved by the present invention is then to provide a device for sampling the smell (and more in general the mixture of volatile compounds) and a related method allowing to obviate the drawbacks mentioned above with reference to the known art.

Such problem is solved by a device according to claim 1 and by a method according to claim 22.

Preferred features of the present invention are subject of the depending claims.

In the present context, under "sample" of a food product one or more portions or substances extracted from the latter are meant, in particular organic volatile substances and still more particularly aromatic substances.

The present invention provides some important advantages. The main advantage consists in that it allows extracting a sample of food product in a highly reproducible way.

Furthermore, the information content of the collected sample is high and at least equal to the one which can be obtained with the above-referred-to traditional procedure. Analogously, the extraction modes result to be minimally invasive and no more invasive than the above-mentioned traditional procedure.

The invention further allows performing an analysis of the sample with objective means and procedures.

The device of the invention can even allow transporting the collected sample under storage conditions.

The device and the method of the invention then allow an effective and efficient extraction of the volatile substances from the samples under examination, particularly in case of matured food products, above all the raw ham.

The device of the invention is compatible with any measuring method downwards the sample collection.

Furthermore, the device results to be extremely robust, above all as the same punch-shaped body able to penetrate the product to sample acts also as receptor of the volatile substances. Such manufacturing in one single piece of the penetration and collection element even allows a greater intrinsic sensitivity of the same device.

Other advantages, features and use modes of the present invention will result evident from the following detailed description of some embodiments, shown by way of example and not with limitative purpose.

### Brief description of the figures

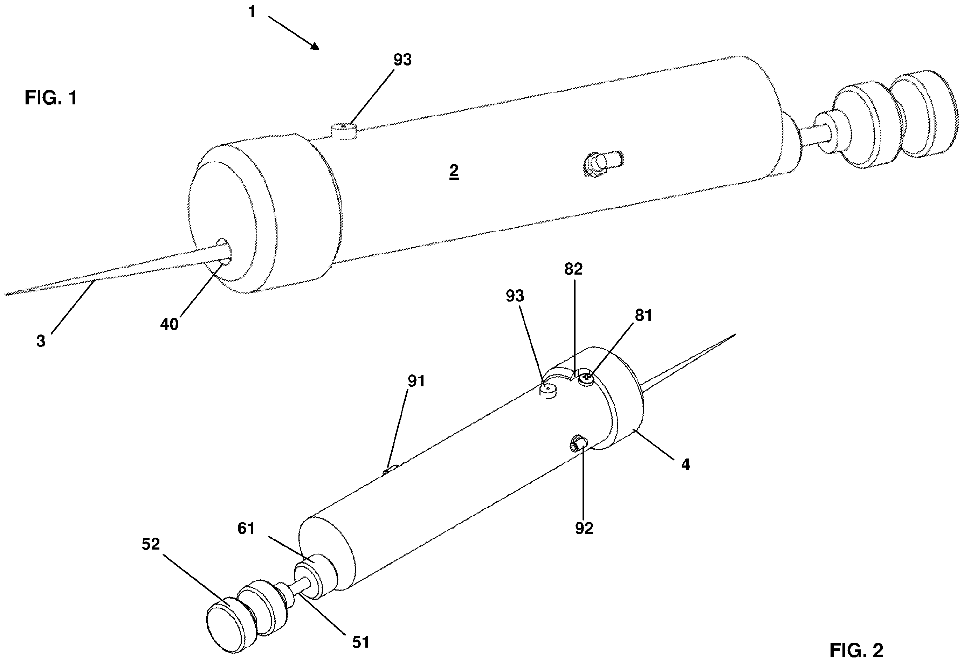

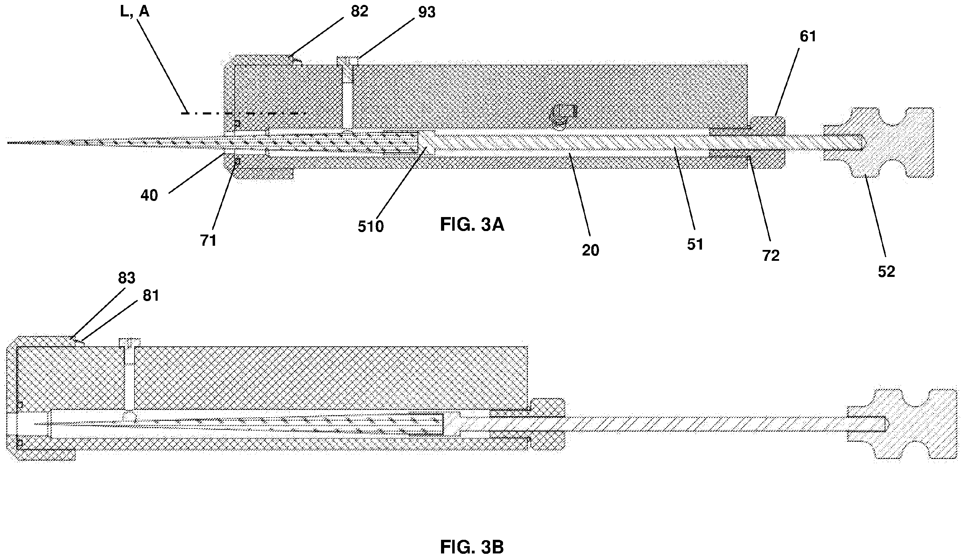

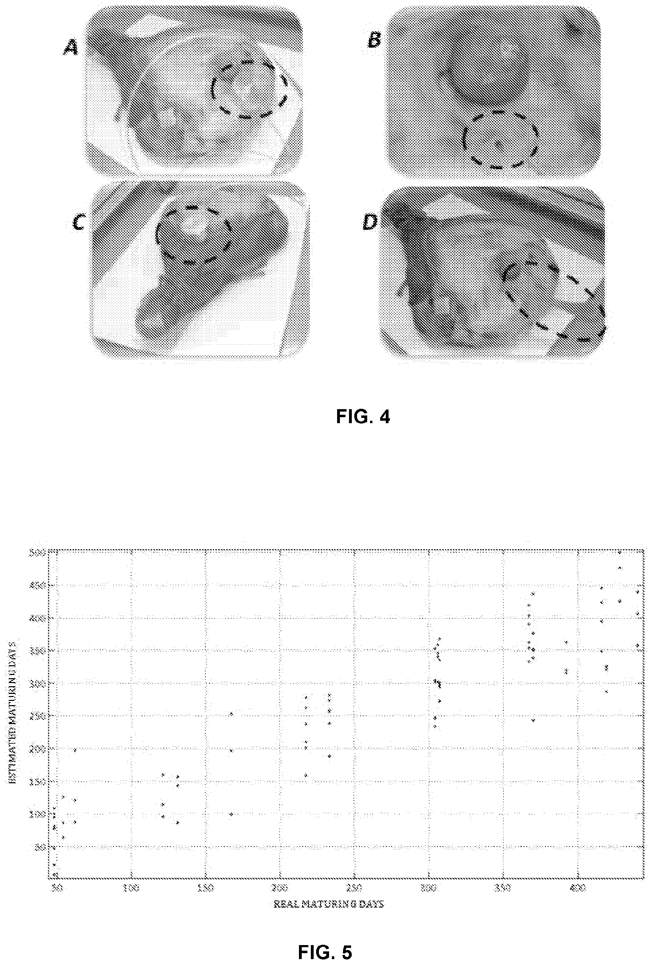

The figures of the enclosed drawings will be referred to, wherein:
▪ Figure 1 shows a front perspective view of a preferred embodiment of the sampling device according to the present invention;
▪ Figure 2 show a back perspective view of the device of Figure 1;
▪ Figures 3A and 3B show, each one, a view in longitudinal section of the device of Figure 1, in a sampling and storage, respectively, configuration of the sample;
▪ Figure 4 shows some ham samples used during experimental tests, the latter partially performed with the device of Figure 1; and
▪ Figure 5 shows a graph related to the carrying out of experimental tests with the device of Figure 1.

### Detailed description of preferred embodiments

With reference to Figures 1, 2, 3A and 3B, a device for sampling a food product according to a preferred embodiment of the invention is designated as a whole with 1.

In the present example, the device 1 is meant for - and it will be described with reference to - checking the maturing of raw ham. It is understood that the same device described herein 1 or embodiments alternative thereto can be used for sampling different, matured or not, food products.

The device 1 first of all comprises a main body 2 with substantially elongated shape. In particular, the main body 2 has a prevailing extension longitudinal direction, designated with L, which in the present example corresponds to that of a symmetry longitudinal axis A of the main body 2 itself.

In the present example, the main body has substantially cylindrical geometry.

The main body 2 has an inner chamber 20, visible in Figure 3A and 3B, extending too substantially along the direction L for the whole length of the main body 2 itself, by crossing it longitudinally from one side to the other one. The chamber 20 has a decentralized arrangement with respect to the symmetry axis A of the main body 2.

As it will be illustrated hereinafter, the chamber 20 is suitable to receive a sample of product and for this reason it will be designated as storage chamber. The main body 2 can be made, for example, of polyoxymethylene (also called Delrin).

The device 1 further comprises a collecting element 3, in particular with a substantially punch-shaped form, suitable to penetrate a food product to collect indeed a sample. The punch-shaped element 3 has a porosity suitable to allow the collection of organic volatile substances, thus acting as receptor for such substances. The collecting element is then implemented in one single piece.

In the present example, the punch-shaped element 3 is at least partly made of horse bone. Embodiment variants can provide the use of polymeric materials and/or different expedients to allow collecting volatile substances.

As it is better visible in Figure 3A and 3B, the punch-shaped element 3 is integral to a stem 51, and in particular coupled by a joint at one shaped terminal end 510 of the latter. In the present example, such shaped end 510 defines a seat wherein a proximal end (that is nearer to the operator during use) of the punch-shaped element 3 is received .

At the end opposite to the one for the connection to the punch-shaped element 3, the stem 51 is made integral, for example by means of a threaded connection, or integral with a knob 52 which can be grasped by an operator to actuate the punch-shaped element 3 according to modes which will be illustrated shortly.

The configuration of the device 1 is so that the assembly stem 51 - punch-shaped element 3 is slidingly connected to the main body 2, resulting to be in particular equipped with a translatory motion substantially in direction L within the chamber 20.

Embodiment variants can provide that the punch-shaped element 3 is removable and/or interchangeable.

At its own proximal longitudinal end, the chamber 20 is closed by means of an element 61, preferably made integral to the wall of the chamber 20 by means of a threaded coupling.

On the opposite side of the chamber 20, that is at the end which could be defined distal as farer from the operator during use, the device 1 provides means for the selective opening and closing of the chamber 20, designated with 4. In the present example the means 4 is implemented under the shape of a plug with cylindrical geometry coupled with the outer surface of the main body 2 by means of a threaded connection.

The preferred configuration is so that the opening/closing means 4 remains integral to the main body 2 with respect to the translations, but a rotation of the means itself is allowed around an axis with substantially L-like direction and preferably coincident with A. The extent of the rotation is limited by means of stopping means, implemented in the present example by means of a post or screw 81. Such stopping means is suitable to stop in abutment the opening/closing means 4 at corresponding profiles or shaped edges of the latter. In particular - and as it will be better explained even with reference to the operating modes of the device 1 - the means 4 provides a first profile or abutment edge 82, visible in Figure 2 and in Figure 3A, corresponding to an opening configuration of the chamber 20, and a second profile or abutment edge 83, visible in Figure 3B, corresponding to a closing configuration of the chamber 20.

To the purpose of the above-mentioned opening/closing of the chamber 20, the means 4 bears an eccentric hole 40, arranged so as to allow indeed, by means of a selective rotation of the means 4 itself, the opening and/or closing of the chamber 20. In particular, according to the rotation direction of the means 4, the hole 40 arranges at the entrance of the chamber 20, by opening it, or far away therefrom, by determining the closing thereof by the means 4 itself.

Both at the means 4, and in particular the hole 40, and at the element 61 sealing means is preferably provided, for example gaskets, to guarantee the sealing, or hermetic, closure of the storage chamber 20. In Figure 3A and 3B such sealing means is represented under the form of o-rings and designated with 71 and 72, respectively.

In the present embodiment, the device 1 further comprises means for the connection with a measuring apparatus or device, which in the present example is arranged at the main body 2 so as to result to be in communication with the storage chamber 20. Preferably, such means comprises one or more two-way valves, designated with 91 and 92 and/or a septum for SPME (*Solid Phase Micro-Extraction*) analysis, designated with 93.

In the present embodiment, two two-way valves 91 and 92, are indeed provided, arranged on the side coating of the main body 2 in diametrally opposite and longitudinally offset positions. Still in the present example, even the septum 93 is arranged onto the side coating of the main body 2. Embodiment variants can provide a different arrangement of the just mentioned elements, for example even at the longitudinal/basis ends of the main body 2.

The valve or each valve 91, 92 is suitable to place into communication the environment of the chamber 20 with a measuring environment bearing detection means suitable to perform measurements/analyses of organic volatile substances, in particular of aroma. Similarly, the septum for SPME analysis, known on itself for the person skilled in the art, is suitable to insert a fibre for the adsorption of the organic volatile substances, according to modes known in the art.

Embodiment variants can provide that specific means for detecting/analyzing organic volatile substances, in particular aromatic ones, for example sensors and/or transducers, is directly arranged, permanently or in a removable way, within the storage chamber 20. In such variants, the device 1 however can or cannot be equipped with said means for connection with a measuring apparatus or device.

The operating modes of the device 1 are illustrated hereinafter.

When an operator wishes to perform a sampling of the ham to verify the level of maturing thereof, he/she grasps the device 1 at the main body 2 and rotates the opening/closing means 4 so as to bring the hole 40 at the entrance of the chamber 20 and the first abutment profile 82 to abut onto the stopping means 81. Then, by actuating the knob 52, and in particular by pushing it, the operator makes the punch-shaped element 3 to protrude from the chamber 20 indeed at the hole 40 and he/she makes it to penetrate the ham as far as a predetermined depth.

Embodiment variants can provide suitable means for designating the penetration depth, associated to the punch-shaped element 3 and/or to the main body 2, and/or means for the automatic actuation of the punch-shaped element 3 as far as a wished/predetermined depth.

The punch-shaped element 3 is then retracted, in the example considered herein still by the operator by means of the knob 52, and brought back within the chamber 20. The operator then rotates the means 4 in the direction producing the closing of the chamber 20 and bringing the second abutment profile 83 to abut onto the stopping means 81. At this point, detections, measurements and analyses of organic volatile substances, and in particular of aroma, can be performed, by connecting the device 1 to outer means by means of valves 91, 92 and/or the septum 93 or, in the above mentioned variant, by using detection means therewith the same device 1 is equipped.

Once ended the measurement procedures the chamber 20 can be cleaned up by means of a flow of inert air by using the valves 91 and 92 for subsequent measurements.

The invention has as subject a method for checking a food product based upon the detection of organic volatile substances, in particular aromatic substances, of a sample of product. Such method is preferably performed by means of the device 1 as sofar described and according to the already illustrated modes.

It will be then appreciated that the device and the method of the invention, differently from the already known techniques, allow not to disperse into the environment the volatile compounds extracted from the sampled product. On the contrary, they are confined in the inner chamber of the main body to be then analyzed by means of any instrument suitable to the analysis of the volatile portion.

By way of example and not with limitative purpose, some tests will be described hereinafter, performed with a prototype of the device 1, used for checking the maturing of raw ham.

The inventors have performed a first assembly of tests aiming at checking the capability of the device to insulate the aroma extracted from the sample of food product from the volatile compounds contained in the maturing environment and constituting the "noise" which could interfere with a correct measurement.

By referring to Figure 4, two different strategies for extracting the aroma were experimented, by using in particular:
(i) a bell made of steel (cases A, B and C of Figure 4) positioned on the surface of the thigh of raw ham, suitable to sample and examine the volatile compounds comprised within the compartment defined between the bell and the thigh itself; samplings in different points of the surface were performed, and specifically in the area surrounding the femur proximal epiphysis with (case B) and without (case A) perforation of the surface, and in an area covered by the skin without perforation of the surface (case C);
(ii) the above-described sampling device 1, used in the area of the traditional procedure, that is in the area surrounding the femur proximal epiphysis (case D).

The above-mentioned types of sampling (A, B, C and D) are summarized in the following table.

| | Sampler | Area | Perforation |
|---|---|---|---|
| A | Steel bell | femur proximal epiphysis | no |
| B | Steel bell | femur proximal epiphysis | yes |
| C | Steel bell | portion covered by skin | no |
| D | FLUTE | femur proximal epiphysis | yes |

Then the statistical test MANOVA (*Multivariate ANalysis Of VAriance*) was performed, allowing establishing the real link between the performed measurements. The null hypothesis (that is the one to be verified) provided that the data obtained with the adopted sampling techniques (A, B, C and D) did not show significant differences with respect to the background, that is to the air of the measurement environment, and that the small differences, even if existing, were due only to an intrinsic noise, typical of the experimental measurements, and not to features which the samples really had.

The subsequent table shows the results of the MANOVA performed on the above-illustrated different types of sampling.

| Method | MANOVA test *p₀* | Hypothesis *H₀* (environmental air) |
|---|---|---|
| A | 0.054 | accepted |
| B | 0.0126 | rejected |
| C | 0.0087 | rejected |
| D | 9.65 x 10⁻¹¹ | rejected |

By analyzing the results illustrated in the table, it results clear that the best sampling method is the one performed by means of the sampling device 1 in the area surrounding the femur proximal epiphysis. From the obtained results it can be even deduced that the device 1 guarantees a high representativity of the measurements, by insulating in an effective way the volatile compounds released by the sample by the background air contained in the measurement environment.

In a second test assembly one wanted to check the representativity and the significance of the measurements performed by means of the device 1, by processing the data obtained from the analyses performed on samples of raw ham with the purpose of extracting information about the provenance and the degree of maturing of the sampled examples.

The obtained results can be divided into two families:
▪ measurement of the maturing level (in days);
▪ classification of the maturing steps and of the provenance breedings.

The analyses were performed based upon data of the following tables.

| Provenance Breeding | Number of Samples | Maturing Degree |
|---|---|---|
| | 3 | 48 |
| | 6 | 48 |
| BASSIANO Breeding placed near the city of Bassiano | 3 | 54 |
| | 3 | 62 |
| | 3 | 131 |
| | 3 | 167 |
| | 6 | 217 |
| | 6 | 233 |
| | 6 | 304 |
| | 4 | 306 |
| | 6 | 307 |
| | 6 | 367 |
| | 6 | 370 |
| | 3 | 392 |
| | 6 | 416 |
| | 3 | 419 |
| | 6 | 428 |
| | | |

| Provenance Breeding | Number of Samples | Maturing Degree |
|---|---|---|
| | 6 | 24 |
| | 3 | 48 |
| | 3 | 62 |
| | 2 | 101 |
| | 2 | 111 |
| CLAI Cooperativ a Lavoraton Agricoli Imolesi breeding placed in Imola | 2 | 129 |
| | 3 | 139 |
| | 3 | 167 |
| | 2 | 185 |
| | 2 | 195 |
| | 2 | 213 |
| | 6 | 216 |
| | 6 | 241 |
| | 2 | 270 |
| | 2 | 280 |
| | 6 | 293 |
| | 4 | 294 |
| | 2 | 298 |
| | 6 | 367 |
| | 2 | 371 |
| | 3 | 376 |
| | 3 | 381 |
| | 2 | 399 |
| | 6 | 413 |
| | 3 | 421 |
| | 3 | 425 |
| | 3 | 440 |

The graph of Figure 5 shows the results obtained by estimating the maturing days of samples selected from the whole data-set, in particular of the samples coming only from BASSIANO breeding.

The errors of estimating models can be considered acceptable above all by considering that in the practice it is not necessary, nor useful, estimating the maturing days with a high precision: in a maturing process lasting 15-18 months it is not probable that checks on the maturing degree are performed with rates lower than few tens of days.

Furthermore, in a considerably long productive process, the fact of estimating the maturing degree of a sample with an error, for example, of few tens of days, does not guarantee a relevant improvement in terms of maturing and therefore it does not guarantee qualitative standards.

Moreover the significance of the measurements with respect to the fundamental parameters of the subject of the analyses appears clear: the use of the device 1 allows monitoring the main interest quantity and that is the variations in concentration of VOCs (*Volatile Organic Compounds*) of the raw ham due to the maturing process.

Analyses on four different types of classification were performed:
▪ classification of 6 maturing steps;
▪ classification of 6 *
▪ maturing steps with samples coming from the same breeding;
▪ classification of the samples with respect to the provenance breeding inside the single steps;
▪ classification of the samples with respect to the provenance breeding on the whole maturing (samples from 24 to 440 days).

The present invention has been sofar described with reference to preferred embodiments. It is to be meant that other embodiments may exist, all belonging to the same inventive core, as defined by the protection scope of the herebelow reported claims.

## Claims

1. A device (1) for sampling a food product, which device (1) is apt to allow the detection of volatile substances and comprises: a punch-shaped collecting element (3), suitable to penetrate a food product for collecting a sample thereof; said punch-shaped element (3) being made of one single piece and having a porosity suitable to allow the collection of organic volatile substances, **characterised in that** the device further comprises a main body (2) having a chamber (20) for storing a sample of product,wherein the collecting element (3) is connected to said main body (2) and movable with respect to the latter between a first collecting position, in which it protrudes from said storage chamber (20) for collecting the sample, and a second storage position, in which it is housed in said chamber (20); and means (4) for the selective opening and closing of said storage chamber (20), apt to allow protrusion of said collecting element (3); and wherein, upon use, the volatile substances are confined in said chamber (20) for storing said main body (2) to be then analyzed by means of a suitable instrument.

2. The device (1) according to claim 1, wherein said main body (2) has a substantially elongated shape, preferably with a substantially cylindrical geometry.

3. The device (1) according to anyone of the preceding claims, wherein said storage chamber (20) is obtained eccentrically inside said main body (2).

4. The device (1) according to anyone of the preceding claims, wherein said collecting element (3) is coupled with said main body (2) at said storage chamber (20), preferably by means of a sealed coupling.

5. The device (1) according to anyone of the preceding claims, wherein said collecting element (3) is slidably coupled with said main body (2), and preferably movable along a direction (L) of longitudinal extension of said body.

6. The device (1) according to anyone of the preceding claims, wherein said collecting element (3) is at least partly made of horse bone.

7. The device (1) according to anyone of the preceding claims, wherein the overall configuration is such that said collecting element (3) is removable and/or interchangeable.

8. The device (1) according to anyone of the preceding claims, wherein said means (4) for the selective opening and closing is actuatable in rotation for producing an opening and/or closing of said storage chamber (20).

9. The device (1) according to anyone of the preceding claims, wherein said opening and closing means comprises closing means (4) bearing a hole (40), the latter suitable to be arranged at said storage chamber (20) for allowing protrusion of said collecting element (3) through the hole.

10. The device (1) according to the preceding claim, wherein said hole (40) is obtained eccentrically on said closing element (4).

11. The device (1) according to claim 9 or 10, wherein said closing element (4) can be actuated in rotation to bring said hole (4) to arrange at said storage chamber (20).

12. The device (1) according to anyone of the preceding claims, comprising means (81-83) for stopping said opening and closing means (4), which stopping means (81-83) defines an opening position and a closing position of said storage chamber (20).

13. The device (1) according to anyone of the preceding claims, comprising means (51, 52) for moving said collecting element (3), actuatable by a user to move said collecting element (3) between said collecting and storage positions.

14. The device (1) according to the preceding claim, wherein said moving means comprises a stem (51) slidable inside said storage chamber (20) and connected to, or integral with, said collecting element (3), preferably at one end of said stem.

15. The device (1) according to the preceding claim, wherein said collecting element (3) is held by a joint at the end of said stem (51).

16. The device (1) according to anyone of the preceding claims, comprising sealing means (71, 72) suitable to ensure a hermetic seal of said storage chamber (20).

17. The device (1) according to anyone of the preceding claims, comprising means (91, 92, 93) for connecting with a measuring device, preferably arranged at said main body (2), which means preferably comprises one or more two-way valves (91, 92) and/or a septum (93) for SPME (*Solid Phase Micro-Extraction*) analysis.

18. The device (1) according to any one of the preceding claims, which is a device for checking the maturing of the food product, in particular of raw ham.

19. An assembly for measuring volatile substances, in particular aromatic ones, comprising a sampling device (1) according to anyone of the preceding claims and detection means - in particular sensors and/or transducers - of volatile substances present in said storage chamber (20) and/or on said collecting element (3).

20. The measuring assembly according to the preceding claim, wherein said detection means is permanently or removably arranged inside said storage chamber (20).

21. A method for checking a food product based upon the detection of volatile substances, in particular aromatic ones, of a sample of product, envisaging the steps of:
- providing a punch-shaped collecting element (3), suitable to penetrate a food product for collecting a sample thereof, which collecting element (3) is connected to a main body (2) bearing a storage chamber (20) for storing a sample of product, which collecting element (3) is movable with respect to said main body (2) between a first collecting position, in which it protrudes from said storage chamber (20) for collecting the sample, and a second storage position, in which it is housed in said chamber (20);
- collecting the sample of volatile substances by penetration of said collecting element (3) into the product;
- arranging said collecting element (3) bearing the volatile substances in said storage chamber (20), so that said volatile substances are confined in said storage chamber (20); and
- performing a detection of volatile substances present in said storage chamber (20) and/or on said collecting element (3),
wherein said punch-shaped element (3) is made of one single piece and has a porosity suitable to allow collecting organic volatile substance.

22. The method according to the preceding claim, which is a method for checking the maturing of a food product, in particular raw ham.

23. The method according to claim 21 or 22, which uses a sampling device (1) and/or a measuring assembly according to anyone of the claims 1 to 20.

## Patentansprüche

1. Vorrichtung (1) zum Entnehmen einer Probe eines Nahrungsmittelproduktes, wobei die Vorrichtung (1) geeignet ist, die Erfassung flüchtiger Substanzen zu ermöglichen, und umfasst: ein stanznadelförmiges Aufnahmeelement (3), das geeignet ist, in ein Nahrungsmittelprodukt einzudringen, um davon eine Probe zu nehmen; wobei das stanznadelförmige Aufnahmeelement (3) aus einem einzigen Stück gefertigt ist und eine Porosität aufweist, die geeignet ist, die Aufnahme organischer flüchtiger Substanzen zu ermöglichen, **dadurch gekennzeichnet, dass** die Vorrichtung weiterhin ein Hauptgehäuse (2) umfasst, das eine Kammer (20) zum Speichern einer Probe des Produktes aufweist, wobei das Aufnahmeelement (3) mit dem Hauptgehäuse (2) verbunden und bezüglich letzterem zwischen einer ersten Aufnahmeposition, in der es aus der Speicherkammer (30) zum Aufnehmen der Probe vorsteht, und einer zweiten Speicherposition, in der es in der Kammer (20) aufgenommen ist, bewegbar ist; und Mittel (4) zum selektiven Öffnen und Schließen der Speicherkammer (20), geeignet, um ein Vorstehen des Aufnahmeelementes (3) zu ermöglichen; und wobei unter Verwendung die flüchtigen Substanzen in der Kammer (20) zum Speicher in dem Hauptgehäuse (2) eingeschlossen sind, um sie dann mittels eines geeigneten Instrumentes zu analysieren.

2. Vorrichtung (1) nach Anspruch 1, wobei das Hauptgehäuse (2) eine im Wesentlichen längliche Form hat, vorzugsweise mit einer im Wesentlichen zylindrischen Geometrie.

3. Vorrichtung (1) nach einem der vorhergehenden Ansprüche, wobei die Speicherkammer (20) exzentrisch innerhalb des Hauptgehäuses (2) verfügbar ist.

4. Vorrichtung (1) nach einem der vorhergehenden Ansprüche, wobei das Aufnahmeelement (3) mit dem Hauptgehäuse (2) an der Speicherkammer (20) verbunden ist, vorzugsweise mittels einer gedichteten Kupplung.

5. Vorrichtung (1) nach einem der vorhergehenden Ansprüche, wobei das Aufnahmeelement (3) mit dem Hauptgehäuse (2) gleitend gekoppelt ist und vorzugsweise entlang einer Richtung (L) longitudinaler Erstreckung des Gehäuses bewegbar ist.

6. Vorrichtung (1) nach einem der vorhergehenden Ansprüche, wobei das Aufnahmeelement (3) wenigstens zum Teil aus Pferdeknochen gefertigt ist.

7. Vorrichtung (1) nach einem der vorhergehenden Ansprüche, wobei die Gesamtkonfiguration derart ist, dass das Aufnahmeelement (3) entfernbar und/oder austauschbar ist.

8. Vorrichtung (1) nach einem der vorhergehenden Ansprüche, wobei die Mittel (4) zum selektiven Öffnen und Schließen drehbar bewegbar sind, um ein Öffnen und/oder Schließen der Speicherkammer (20) zu erzeugen.

9. Vorrichtung (1) nach einem der vorhergehenden Ansprüche, wobei die Öffnungs- und Schließmittel Schließmittel (4) umfassen, die ein Loch (40) beherbergen, wobei letzteres geeignet ist, an der Speicherkammer (20) angeordnet zu werden, um ein Vorstehen des Aufnahmeelementes (3) durch das Loch zu ermöglichen.

10. Vorrichtung (1) nach einem der vorhergehenden Ansprüche, wobei das Loch (40) exzentrisch an dem Schließelement (4) verfügbar ist.

11. Vorrichtung (1) nach Anspruch 9 oder 10, wobei das Schließelement (4) drehbar bewegbar ist, um das Loch (4) in eine Anordnung an der Speicherkammer (20) zu bringen.

12. Vorrichtung (1) nach einem der vorhergehenden Ansprüche, die Mittel (81-83) zum Stoppen der Öffnungs- und Schließmittel (4) umfasst, wobei die Stoppmittel (81-83) eine Öffnungsposition und eine Schließposition der Speicherkammer (20) definieren.

13. Vorrichtung (1) nach einem der vorhergehenden Ansprüche, die Mittel (51,52) zum Bewegen des Aufnahmeelementes (3) umfasst, welche durch einen Nutzer bewegbar sind, um das Aufnahmeelement (3) zwischen der Aufnahme- und der Speicherposition zu bewegen.

14. Vorrichtung (1) nach einem der vorhergehenden Ansprüche, wobei die Bewegungsmittel einen Schaft (51) gleitfähig innerhalb der Speicherkammer (20) und in Verbindung mit oder einstückig mit dem Aufnahmeelement (3) umfassen, vorzugsweise an einem Ende des Schaftes.

15. Vorrichtung (1) nach einem der vorhergehenden Ansprüche, wobei das Aufnahmeelement (3) durch eine Verbindung an dem Ende des Schaftes (51) gehalten wird.

16. Vorrichtung (1) nach einem der vorhergehenden Ansprüche, welche Dichtmittel (71, 72) umfasst, die geeignet sind, eine hermetische Abdichtung der Speicherkammer (20) sicherzustellen.

17. Vorrichtung (1) nach einem der vorhergehenden Ansprüche, die Mittel (91, 92, 93) zum Verbinden mit einem Messgerät umfasst, vorzugsweise an dem Hauptgehäuse (2) angeordnet, wobei die Mittel vorzugsweise ein oder mehr Zweiwegeventile (91, 92) und/oder ein Septum (93) zur SPME-Analyse (Festphasenmikroextraktion) umfassen.

18. Vorrichtung (1) nach einem der vorhergehenden Ansprüche, welche eine Vorrichtung zum Überprüfen der Reifung des Nahrungsmittelproduktes ist, vorzugsweise von rohem Schinken.

19. Anordnung zum Messen flüchtiger Substanzen, insbesondere aromatischer, die eine Probevorrichtung (1) nach einem der vorhergehenden Ansprüche und Erfassungsmittel - insbesondere Sensoren und/oder Transducer - für flüchtige Substanzen umfasst, die in der Speicherkammer (20) und/oder an dem Aufnahmeelement (3) anwesend sind.

20. Messanordnung nach dem vorhergehenden Anspruch, wobei die Erfassungsmittel permanent oder entfernbar innerhalb der Speicherkammer (20) angeordnet sind.

21. Verfahren zum Überprüfen eines Nahrungsmittelproduktes basierend auf der Erfassung flüchtiger Substanzen, insbesondere aromatischer, einer Probe des Produktes, welches die folgenden Schritte vorsieht:
- Bereitstellen eines stanznadelförmigen Aufnahmeelementes (3), das geeignet ist, in ein Nahrungsmittelprodukt zum Entnehmen einer Probe desselben einzudringen, wobei das Aufnahmeelement (3) mit einem Hauptgehäuse (2) verbunden ist, der eine Speicherkammer (20) zum Speichern einer Probe des Produktes beherbergt, wobei das Aufnahmeelement (3) bezüglich des Hauptgehäuses (2) zwischen einer ersten Aufnahmeposition, in der es von der Speicherkammer (20) zum Entnehmen der Probe vorsteht, und einer zweiten Speicherposition, in der es in der Kammer (20) aufgenommen ist, bewegbar ist;
- Entnehmen der Probe flüchtiger Substanzen durch Einführen des Aufnahmeelementes (3) in das Produkt;
- Anordnen des Aufnahmeelementes (3), das die flüchtigen Substanzen trägt, in der Speicherkammer (20), so dass die flüchtigen Substanzen in der Speicherkammer (20) eingeschlossen werden; und
- Durchführen einer Erfassung flüchtiger Substanzen, die in der Speicherkammer (20) und/oder an dem Aufnahmeelement (3) anwesend sind,
wobei das stanznadelförmige Element (3) aus einem einzigen Stück gefertigt ist und eine Porosität aufweist, die es ermöglicht, organische flüchtige Substanzen aufzunehmen.

22. Verfahren nach dem vorhergehenden Anspruch, welches ein Verfahren zum Überprüfen der Reifung eines Nahrungsmittelproduktes ist, insbesondere von rohem Schinken.

23. Verfahren nach Anspruch 21 oder 22, welches eine Probevorrichtung (1) und/oder eine Messanordnung nach einem der Ansprüche 1 bis 20 nutzt.

## Revendications

1. Dispositif (1) pour échantillonner un produit alimentaire, lequel dispositif (1) peut permettre la détection de substances volatiles et comprend : un élément de collecte en forme de poinçon (3) approprié pour pénétrer dans un produit alimentaire afin de collecter un échantillon de celui-ci ; ledit élément en forme de poinçon (3) étant réalisé d'un seul tenant et ayant une porosité qui est appropriée pour permettre la collecte de substances volatiles organiques, **caractérisé en ce que** le dispositif comprend en outre un corps principal (2) comprenant une chambre (20) pour stocker un échantillon de produit, dans lequel l'élément de collecte (3) est relié au dit corps principal (2) et est mobile par rapport à ce dernier entre une première position de collecte, où il fait saillie à partir de ladite chambre de stockage (20) pour collecter l'échantillon, et une seconde position de stockage, où il est logé dans ladite chambre (20) ; et des moyens (4) pour l'ouverture et la fermeture sélective de ladite chambre de stockage (20), pouvant permettre au dit élément de collecte (3) de faire saillie ; et dans lequel, à l'utilisation, les substances volatiles sont confinées dans ladite chambre (20) pour stocker ledit corps principal (2) devant être ensuite analysé au moyen d'un instrument approprié.

2. Dispositif (1) selon la revendication 1, dans lequel ledit corps principal (2) a une forme essentiellement élongée, avec une géométrie essentiellement cylindrique de préférence.

3. Dispositif (1) selon l'une quelconque des revendications précédentes, dans lequel ladite chambre de stockage (20) est obtenue de manière excentrique à l'intérieur dudit corps principal (2).

4. Dispositif (1) selon l'une quelconque des revendications précédentes, dans lequel ledit élément de collecte (3) est couplé au dit corps principal (2) au niveau de ladite chambre de stockage (20), de préférence au moyen d'un couplage d'étanchéité.

5. Dispositif (1) selon l'une quelconque des revendications précédentes, dans lequel ledit élément de collecte (3) est couplé de manière coulissante audit corps principal (2) et mobile de préférence le long d'une direction (L) d'extension longitudinale dudit corps.

6. Dispositif (1) selon l'une quelconque des revendications précédentes, dans lequel ledit élément de collecte (3) est réalisé au moins partiellement à partir d'os de cheval.

7. Dispositif (1) selon l'une quelconque des revendications précédentes, dans lequel la configuration d'ensemble est telle que l'élément de collecte (3) est amovible et/ou interchangeable.

8. Dispositif (1) selon l'une quelconque des revendications précédentes, dans lequel lesdits moyens (4) utilisés pour l'ouverture et la fermeture sélective peuvent être actionnés en rotation pour entraîner l'ouverture et/ou la fermeture de ladite chambre de stockage (20).

9. Dispositif (1) selon l'une quelconque des revendications précédentes, dans lequel lesdits moyens d'ouverture et de fermeture comprennent un moyen de fermeture (4) supportant un orifice (40), ce dernier étant approprié pour être disposé au niveau de ladite chambre de stockage (20) pour permettre au dit élément de collecte (3) de faire saillie à travers l'orifice.

10. Dispositif (1) selon l'une quelconque des revendications précédentes, dans lequel ledit orifice (40) est obtenu de manière excentrique sur ledit élément de fermeture (4).

11. Dispositif (1) selon la revendication 9 ou 10, dans lequel ledit élément de fermeture (4) peut être actionné en rotation pour amener ledit orifice (40) à se disposer au niveau de ladite chambre de stockage (20).

12. Dispositif (1) selon l'une quelconque des revendications précédentes, comprenant des moyens (81-83) destinés à arrêter lesdits moyens d'ouverture et de fermeture (4), lesdits moyens d'arrêt (81-83) définissant une position d'ouverture et une position de fermeture de ladite chambre de stockage (20).

13. Dispositif (1) selon l'une quelconque des revendications précédentes, comprenant des moyens (51-52) pour déplacer ledit élément de collecte (3), pouvant être actionnés par un utilisateur pour déplacer ledit élément de collecte (3) entre lesdites positions de collecte et de stockage.

14. Dispositif (1) selon la revendication précédente, dans lequel lesdits moyens de déplacement comprennent une tige (51) pouvant coulisser à l'intérieur de ladite chambre de stockage (20) et reliée, ou d'un seul tenant, au dit élément de collecte (3), de préférence à une extrémité de ladite tige.

15. Dispositif (1) selon la revendication précédente, dans lequel ledit élément de collecte (3) est maintenu par un joint à l'extrémité de ladite tige (51).

16. Dispositif (1) selon l'une quelconque des revendications précédentes, comprenant des moyens d'étanchéité (71-72) appropriés pour assurer un scellage hermétique de ladite chambre de stockage (20).

17. Dispositif (1) selon l'une quelconque des revendications précédentes, comprenant des moyens (91, 92, 93) pour une connexion à un dispositif de mesure, disposé de préférence au dit corps principal (2), lesquels moyens comprennent de préférence une ou plusieurs soupapes bidirectionnelles (91, 92) et/ou un septum (93) pour une analyse SPME (micro-extraction en phase solide).

18. Dispositif (1) selon l'une quelconque des revendications précédentes, qui consiste en un dispositif pour contrôler le degré de maturation du produit alimentaire, en particulier du jambon cru.

19. Ensemble destiné à mesurer des substances volatiles, notamment des substances aromatiques, comprenant un dispositif d'échantillonnage (1) selon l'une quelconque des revendications précédentes et des moyens de détection, notamment des capteurs et/ou des transducteurs, de substances volatiles présentes dans ladite chambre de stockage (20) et/ou sur ledit élément de collecte (3).

20. Ensemble de mesure selon la revendication précédente, dans lequel lesdits moyens de mesure sont disposés de manière permanente ou amovible à l'intérieur de ladite chambre de stockage (20).

21. Procédé de contrôle d'un produit alimentaire basé sur la détection de substances volatiles, notamment des substances aromatiques, d'un échantillon de produit, envisageant les étapes consistant à :
- fournir un élément de collecte en forme de poinçon (3), approprié pour pénétrer dans un produit alimentaire afin de collecter un échantillon de celui-ci, lequel élément de collecte (3) est relié au dit corps principal (2) supportant une chambre de stockage (20) pour stocker un échantillon du produit, lequel élément de collecte (3) est mobile par rapport au dit corps principal (2) entre une première position de collecte, où il fait saillie à partir de ladite chambre de stockage (20) pour collecter l'échantillon, et une seconde position de stockage, où il est logé dans ladite chambre (20) ;
- collecter l'échantillon de substances volatiles par la pénétration dudit élément de collecte (3) à l'intérieur du produit ;
- disposer ledit élément de collecte (3) portant les substances volatiles dans ladite chambre de stockage (20), de sorte que lesdites substances volatiles soient confinées dans ladite chambre de stockage (20) ; et
- exécuter une détection de substances volatiles présentes dans ladite chambre de stockage (20) et/ou ledit élément de collecte (3),
dans lequel ledit élément en forme de poinçon (3) est réalisé d'un seul tenant et a une porosité appropriée pour permettre la collecte de substances volatiles organiques.

22. Procédé selon la revendication précédente, qui consiste en un procédé destiné à contrôler le degré de maturation d'un produit alimentaire, notamment de jambon cru.

23. Procédé selon la revendication 21 ou 22, qui utilise un dispositif d'échantillonnage (1) et/ou un ensemble de mesure selon l'une quelconque des revendications 1 à 20. * * *
